# EUROPEAN PATENT SPECIFICATION

(11) **EP 1 058 676 B1**
(45) Date of publication and mention of the grant of the patent: **14.05.2003**
(21) Application number: 99915386.9
(22) Date of filing: 26.02.1999
(51) Int. Cl.: C07C 1/00, C07C 45/00, C07C 249/08

(54) **PROCESS FOR PREPARATION OF OXIMES AND RESULTING PRODUCTS**
VERFAHREN ZUR HERSTELLUNG VON OXIMEN UND DIE DARAUS RESULTIERENDEN PRODUKTE
PROCEDE DE PREPARATION D'OXIMES ET PRODUITS RESULTANTS

(30) Priority: 27.02.1998 BR 9800783
(43) Date of publication of application: 13.12.2000
(73) Proprietor: Conselho Nacional de Desenvolvimento Cientifico e Technologico - CNPQ, 70740-901 Brasilia, DF (BR)
(72) Inventor: SEIDL, Peter Rudolf, CEP-22461-060 Riode Janeiro, RJ (BR); DA CUNHA PINTO, Angelo, Leme, CEP-22010-010 Rio de Janeiro, RJ (BR); MENZEL, Arthur Richard, CEP-23575-000 Rio de Janeiro, RJ (BR); PORTELA COUTO, Roberto Ottoni, CEP-22011-000 Rio deJan eiro, RJ (BR)
(74) Representative: van der Kloet-Dorleijn, G.W.F.
(86) International application number: BR9900020
(87) International publication number: WO99043634

(56) References cited:
- GB-A- 2 104 516
- US-A- 4 707 294
- US-A- 5 399 761
- US-A- 5 488 161
- LAM S.K. & TYMAN J.H.P.: "Long Chain Phenols. Part 18. Conversion of Anacardic Acid into Urushiol" JOURNAL OF THE CHEMICAL SOCIETY PERKIN TRANSACTIONS I, 1981, pages 1942-4952, XP000983535
- DAUBEN, W.G. ED. ET AL.: "Organic Reactions, Volume 28" 1982 , JOHN WILEY & SONS, INC. , NEW YORK XP002160713 * Wynberg H. & Meijer, E.W. "The Reimer-Tiemann Reaction", pp. 1-36 *
- DATABASE CAPLUS ON STN, CHEM. ABSTRACTS, (Columbus, Ohio, USA), No. 93:240530, "Polymer used for Production of Bonded Materials", XP002947497 & EP 15761 A2, 17 September 1980
- DATABASE CAPLUS ON STN, CHEM. ABSTRACTS, (Columbus, Ohio, USA), No. 1993:180519, "Extraction of Cardol and Cardanol from Cashew Nutshell Liquid", XP002947496 & JP 05000979 A (08-01-1991).
- DATABASE CAPLUS ON STN, CHEM. ABSTRACTS, (Columbus, Ohio, USA), No. 1988:77578, "Quantitative Determination of Cardanol Constituents by CGC", XP002947495 & COMMUN., Vol. 10, No. 10, October 1987, pp. 576-578.

## Description

Family of cardanol derivatives products (alkyl hydroxy aryl ketoximes and alkyl hydroxy aryl aldoximes), with complexing properties for di and trivalent metallic cations, which could be applied in conventional liquid-liquid extraction process, and in ionic flotation process, to promote selective separation of metallic cations, transition elements and rare earths.

Oximes are generally produced by reaction of carbonilated compounds, such as aldehydes and ketones, with hydroxylamine, usually generated from hydroxylamine salt, hydroxylamine sulphate or hydroxylamine hydrochloride.

The reaction process is carried out under conditions of strong stirring with hydroxylamine salt in aqueous medium. Such methods, usually require a long time to reaction completion, particularly when oximes includes aromatic groups.

### STATE OF THE ART

Many classes of organic substances behave as excellent metal complexants, nevertheless, many of them, apart of presenting low solubility in water, become completely soluble when complexed with metals, turning impossible their application on solvent extraction process keeping untransfered to the organic phase.

This problem may be solved, through the utilization of complexing substances functionalized by groups that increase their hydroxyphobicity, such as long chain hydrocarbons, linear or branched.

Organic compounds belonging to the class of 7 - alkyl - hydroxyquinolines (oxines) known as Kelex®, and aldoximes or ketoximes (oximes), known as Lix®, are examples of compounds able to act as metal complexants.

The complexing power of these substances with different metals, representative and transition elements and rare earths, let them occupy an important place as extractants in a recovery process.

### FUNDAMENTALS OF THE INVENTION

The present invention has the objective to prepare oximes, which are compounds able to act as extractants of metals, rare earths and other elements, present in hydrometallurgic fluids, where the intention is the separation or enrichment of such element group of elements.

The prepared compounds are applied in solvent extraction process and/or ionic flotation for recovery of gallium, rare earths and other metals.

Solvent extraction is one of the most modern techniques for recovery and purification of metals, in the scope of extractive metallurgy. Oximes have high chelation power for a large number of different metals, apart of other elements (representative, transition and rare earths). This property made that these classes of substances occupy an important place as extractants in metals recovery process, starting from its main sources.

Metals as gallium, rare earths, germanium, etc. obtained with high degree of purity, are used as raw materials in the preparation of high valuable materials, for noble metal utilization such as: semiconductors, superconductors, special alloys for aviation, catalysts, etc.

### INVENTION DEVELOPMENT

The novelty of this invention is the utilization of a vegetal raw material available in Brasil: cashew nut shell liquid (CNSL), a byproduct of cashew's industry.

Cashew nut shell liquid is constituted of a mixture of alkylated phenols, the cardanol, the cardol, the methyl cardol and the anacardic acid.

It is known that cardanol is present in CNSL, in a concentration of 5 to 10%, weight by weight, as mentioned by literature. On the other hand, it is also known that heating CNSL to 200°C, promotes the anacardic acid decarboxylation, its main constituent, transforming it in cardanol. Fractional distillation under reduced pressure, at temperature of 200°C, produces cardanol with yields up to 65% in weight, related to CNSL.

Being cardanol a natural alkylated phenol, it can be used as starting intermediate to obtain aldehyde, which will be submitted to reaction with hydroxylamine, and transformed in oxime, that, like some commercial products, has wide use as extractant for metals, rare earths and other transition elements.

### EXAMPLES

In laboratory, the cashew nut shell liquid was distilled at reduced pressure, in the range of 200 to 230°C. In a round bottom glass reactor with three openings, adapted to a refluxer condensator, addition funnel with pressure equalizator and magnetic stirrer, were added, at inert atmosphere (N₂), 45 g of chloroform and 100 g of cardanol (produced by distillation of CNSL). The system was heated and 300 ml of sodium hydroxyde solution 4,6N, were added, drop by drop, keeping on stirring and refluxing during six hours and thirty minutes, at 65°C. After reaction, the system was allowed to cool at room temperature. Pure hydrochloric acid was added to the reaction mixture until pH = 1. The organic layer was separated from the aqueous layer by decantation. 25 g of hydroxylamine hydrochloride dissolved in 50 ml of water was added to the organic layer, keeping the system at 65°C under agitation, for three hours and thirty minutes. The organic layer was separated from the aqueous layer by decantation. The product was purified by extraction with isopropyl ether and concentrated by evaporation.

The product obtained has shown the following complexing power: 46 mg Cu/g.

### SYNTHESIS OF OXIMES

### Synthesis of alkyl - salicyl - aldehyde, starting from cardanol (constituent of CNSL) through Reimer-Tiemann reaction.

By this reaction phenolic aldehydes are obtained, starting from phenols. Treatment of phenol with chloroform and aqueous solution of sodium hydroxyde, attaches aldehyde group (-CHO), to the aromatic ring, generally at position ortho related to the hydroxyl (-HO).

### Synthesis of oxime, starting from alkyl - salicyl - aldehyde.

Hydroxylamine (NH₂OH) reacts with the group of aldehydes and ketones, generating products which has a double bond between carbon and nitrogen, known as oximes (C=N-OH). Depending on the group of origin, it is possible to prepare aldoximes (from aldehyde) or ketoximes (from ketones).

## Claims

1. A process for the preparation of aldoximes, mainly of formula 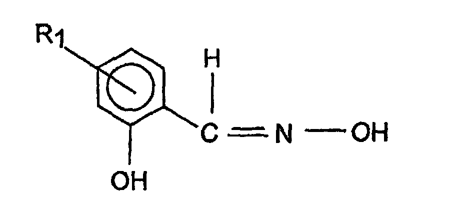 wherein R = C₁₅H₃₁₋ₙ wherein n = 0, 2, 4, 6,
by reaction of a cardanol containing mixture with aqueous sodium hydroxide at a temperature of 20° to 70°C in the presence of chloroform to obtain alkyl salicyl aldehydes, mainly of formula 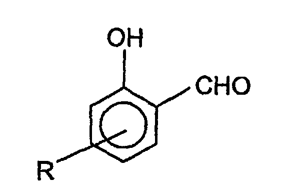 wherein R = C₁₅H₃₁₋ₙ wherein n = 0, 2, 4, 6,
whereafter said alkyl salicyl aldehydes are reacted with hydroxylamine or a salt thereof to obtain said aldoximes,
wherein said cardanol containing mixture is obtained by subjecting cashew nut shell liquid to a heat treatment at a temperature of at least 200°C, under reduced pressure.

2. A process according to claim 1, wherein said heat treatment is effected at a temperature in the range of 200° to 230°C.

3. A process according to claim 1 or 2, wherein said cardanol is hydrogenated before its conversion into said alkyl salicyl aldehydes.

## Patentansprüche

1. Verfahren zur Herstellung von Aldoximen, hauptsächlich mit der Formel 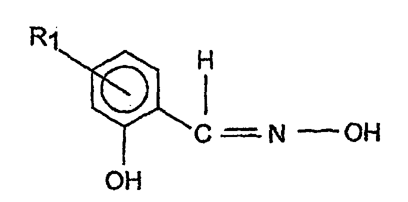 wobei R = C₁₅H₃₁₋ₙ, wobei n = 0, 2, 4, 6,
durch Reaktion eines cardanolhaltigen Gemischs mit Natronlauge bei einer Temperatur von 20 bis 70 °C in Gegenwart von Chloroform unter Bildung von Alkylsalicylaldehyden, hauptsächlich mit der Formel 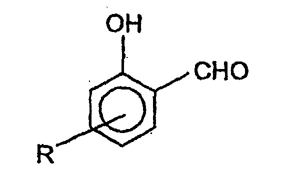 wobei R = C₁₅H₃₁₋ₙ, wobei n = 0, 2, 4, 6,
woraufhin die Alkylsalicylaldehyde mit Hydroxylamin oder einem Salz davon zu den Aldoximen umgesetzt werden, wobei das cardanolhaltige Gemisch erhalten wird, indem man Cashew-Nussschalenflüssigkeit einer Wärmebehandlung bei einer Temperatur von wenigstens 200 °C unter reduziertem Druck unterzieht.

2. Verfahren gemäß Anspruch 1, wobei die Wärmebehandlung bei einer Temperatur im Bereich von 200 bis 230 °C durchgeführt wird.

3. Verfahren gemäß Anspruch 1 oder 2, wobei das Cardanol vor seiner Umwandlung zu den Alkylsalicylaldehyden hydriert wird.

## Revendications

1. Procédé de préparation d'aldoximes, principalement de formule 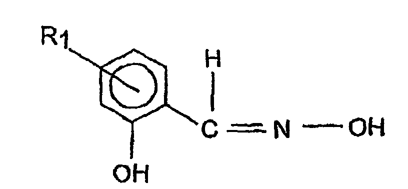 où R = C₁₅H₃₁₋ₙ où n = 0, 2, 4, 6,
par réaction d'un mélange contenant du cardanol avec de l'hydroxyde de sodium aqueux à une température de 20 ° à 70 °C en présence de chloroforme pour obtenir des aldéhydes alkyl-salicyliques, principalement de formule 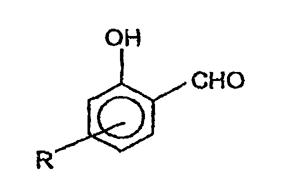 où R = C₁₅H₃₁₋ₙ où n = 0, 2, 4, 6,
après quoi lesdits aldéhydes alkyl-salicyliques sont mis en réaction avec de l'hydroxylamine ou un sel de celui-ci pour obtenir lesdits aldoximes, où ledit mélange contenant du cardanol est obtenu en soumettant de l'huile de noix d'acajou à un traitement thermique à une température d'au moins 200 °C, sous une pression réduite.

2. Procédé selon la revendication 1, où ledit traitement thermique est effectué à une température dans la plage de 200 ° à 230 °C.

3. Procédé selon la revendication 1 ou 2, où ledit cardanol est hydrogéné avant sa conversion en dits aldéhydes alkyl-salicyliques.
